(19) Europäisches Patentamt
European Patent Office
Office européen des brevets

(11) **EP 3 506 827 B1**

(12) **EUROPEAN PATENT SPECIFICATION**

(45) Date of publication and mention
of the grant of the patent:
**20.10.2021 Bulletin 2021/42**

(51) Int Cl.:
***A61B 34/20*** (2016.01)

(21) Application number: **17847197.5**

(86) International application number:
**PCT/US2017/046892**

(22) Date of filing: **15.08.2017**

(87) International publication number:
**WO 2018/044549 (08.03.2018 Gazette 2018/10)**

(54) **RESPIRATION MOTION STABILIZATION FOR LUNG MAGNETIC NAVIGATION SYSTEM**

VORRICHTUNG ZUR STABILISIERUNG EINES MAGNETISCHEN
LUNGENNAVIGATIONSSYSTEM

STABILISATION DE MOUVEMENT DE RESPIRATION POUR SYSTÈME DE NAVIGATION
MAGNÉTIQUE PULMONAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.09.2016 US 201615254141**

(43) Date of publication of application:
**10.07.2019 Bulletin 2019/28**

(73) Proprietor: **Covidien LP
Mansfield, MA 02048 (US)**

(72) Inventors:
• **KOYRAKH, Lev A.
Plymouth
Minnesota 55442 (US)**
• **BARASOFSKY, Ofer
Herzliya 4672559 (IL)**
• **WEINGARTEN, Oren P.
Herzliya 4672559 (IL)**
• **BARAK, Ron
Tel Aviv 6920024 (IL)**

(74) Representative: **Maschio, Antonio
Maschio & Soames IP Limited
30 Carlton Crescent
Southampton SO15 2EW (GB)**

(56) References cited:
**WO-A1-2005/030330     WO-A1-2005/070318
WO-A1-2011/101754     JP-A- 2006 512 950
US-A1- 2009 216 114     US-A1- 2012 172 702
US-A1- 2012 172 712     US-B1- 6 473 635
US-B1- 6 580 938**

**Description**

## BACKGROUND

### 1. Technical Field

The present disclosure provides systems and methods for correcting the detected location of a sensor associated with a medical device in an electromagnetic field by manually or automatically stabilizing a location of the medical device caused by respiration and displaying the stabilized location of the medical device on a display. More particularly, the present disclosure relates to systems and methods for displaying the location of a medical device in a static image or 3D model based on the determined stabilized position of the medical device during medical procedures.

### 2. Discussion of Related Art

When performing a medical procedure, clinicians often rely on patient data including X-ray data, computed tomography (CT) scan data, magnetic resonance imaging (MRI) data, or other imaging data that allows the clinician to view the internal anatomy of a patient. These image data are also utilized to identify targets of interest and to develop strategies for accessing the targets of interest for the surgical treatment. Further, these image data have been used to create a three-dimensional (3D) model of the patient's body to help navigation of the medical device to a target of interest within a patient's body.

Since it is important to treat a target at an exact location from a planned direction, even a small discrepancy between the actual location and an estimated location of the medical device may cause undesired consequences in the medical procedure. Thus, precision in estimating the actual location of the medical device with sufficient level of accuracy is highly desirable during medical procedures.

Further, when the medical device approaches the target following the 3D model, patient's inhaling and exhaling causes medical device to appear to swing in (and possibly out) of the 3D model even though the medical device is stably positioned with respect to internal organs surrounding the target within the patient's body. Thus, stabilizing the respiratory movements for the medical device is also beneficial in properly displaying the location of the medical device during medical procedures.

US 2009/216114 discloses a method and device for real time navigation of a surgical tool handled by an operator in a region of interest of a body itself subject to at least one physiological movement.

US 2012/172702A discloses a system for determining a location of an electrode of a medical device (e.g., a catheter) in a body of a patient. The system includes a localization block for producing an uncompensated electrode location, a motion compensation block for producing a compensation signal (i.e., for respiration, cardiac, etc.), and a mechanism for subtracting the compensation signal from the uncompensated electrode location. The result is a corrected electrode location substantially free of respiration and cardiac artifacts. The motion compensation block includes a dynamic adaptation feature which accounts for changes in a patient's respiration patterns as well as intentional movements of the medical device to different locations within the patient's body. The system further includes an automatic compensation gain control which suppresses compensation when certain conditions, such as noise or sudden patch impedance changes, are detected.

## SUMMARY

The invention is defined in the appended claims.

The present disclosure is directed to systems and methods for stabilizing respiratory movements of a medical device so that the medical device is displayed sufficiently stationary with respect to a static image or model while the patient continuously breathes and the medical device is positioned near a target of interest inside the patient's body.

According to the present disclosure, a system for stabilization based on respiratory movement includes a medical device configured to navigate inside of a patient, a tracking sensor affixed on the medical device and configured to track a location of the medical device, at least one motion sensor located on the patient and configured to sense respiratory movements of the patient, a computer configured to generate a respiratory model based on the respiratory movements sensed by the at least one motion sensor for a predetermined period and to stabilize a location of the medical device based on the respiratory model after the predetermined period, and a display configured to display a graphical representation of the medical device based on the stabilized location on a pre-procedure two-dimensional (2D) image or three-dimensional (3D) model. The computer is further configured to: (i) check (625) an auto-correlation of the tracked locations of the medical device during the predetermined period; and (ii) restart the predetermined period if the tracked locations of the medical device are correlated when a periodic movement exists in the tracked locations of the medical device.

In an aspect, the computer is further configured to receive an instruction to start sampling outputs of the at least one motion sensor and outputs of the tracking sensor for a predetermined period. The computer is further configured to calculate a weight based on the respiratory model and the tracked locations of the medical device, which have been sampled for the predetermined period. A new location of the medical device and new respiratory movement from the at least one motion sensor are sampled at each sampling time for stabilization after the predetermined period. The computer is further configured to multiply the new respiratory movement from the at least one motion sensor with the weight to obtain a reference stabilization signal. The stabilized location of the medical device is obtained by subtracting the reference stabilization signal from the new location of the medical device.

In another aspect, the predetermined period is at least two consecutive respiratory cycles.

In yet another aspect, the respiratory model is based on mean subtracted sampled outputs of the at least one motion sensor for the predetermined period. The respiratory model is generated in matrix representation by performing singular value decomposition on the mean subtracted sampled outputs of the at least one motion sensor for the predetermined period.

The computer is further configured to generate a weight based on the respiratory model and the tracked locations of the medical device if the correlation is less than or equal to the threshold. A new location of the medical device and a new respiratory movement from the at least one motion sensor are sampled at each sampling time for stabilization after the predetermined period. The computer is further configured to multiply the new respiratory movement from the at least one motion sensor with the weight to obtain a reference stabilization signal for the medical device. The stabilized location of the medical device is obtained by subtracting the reference stabilization signal from the new location of the medical device.

In yet another aspect, the respiratory model is generated by performing principal components analysis (PCA). Three principal components are used in the PCA.

In still another aspect, the tracking sensor tracks a location of the distal portion of the medical device. The at least one motion sensor is located on a chest over a lung of the patient.

Any of the above aspects and embodiments of the present disclosure may be combined without departing from the scope of the present disclosure.

## BRIEF DESCRIPTION OF THE DRAWINGS

Objects and features of the presently disclosed system and method will become apparent to those of ordinary skill in the art when descriptions of various embodiments thereof are read with reference to the accompanying drawings, of which:

FIG. 1 is a perspective view of a system for stabilization based on respiratory movements for a medical device in accordance with an embodiment of the present disclosure;

FIG. 2A is a graphical representation illustrating samples, which represent locations of the locatable guide of FIG. 1 caused by respiratory movements;

FIG. 2B is a graphical representation illustrating samples, which represent locations of the motion sensors of FIG. 1 placed around a patient's chest caused by respiratory movements;

FIG. 2C is a graphical representation illustrating principal components of the locations of the motion sensors of FIG. 1;

FIG. 3A is a block diagram for calculating a weight between the samples of a patient sensor triplet (PST) and samples of the medical device in accordance with embodiments of the present disclosure;

FIG. 3B is a block diagram for stabilization based on the respiratory movements for the medical device based on the weight of FIG. 3A in accordance with embodiments of the present disclosure;

FIG. 4 is a graphical representation illustrating samples, which represent locations of the medical device before and after stabilization based on respiratory movements in accordance with an embodiment of the present disclosure;

FIGS. 5A and 5B are flow diagrams illustrating a method for manual stabilization based on respiratory movements for the medical device in accordance with an embodiment of the present disclosure; and

FIG. 6 is a flow diagram illustrating a method for automatic stabilization based on respiratory movements for the medical device in accordance with embodiments of the present disclosure.

## DETAILED DESCRIPTION

The present disclosure provides systems and methods for detecting the location of a sensor associated with a medical device in an electromagnetic field, depicting the location in one or more pre-procedure images or 3D models derived from the pre-procedure images on a display, and manually or automatically stabilizing the location of the medical device by reducing or eliminating movement caused by respiration and displaying the stabilized location of the medical device on a display.

The medical procedures of the present disclosure are generally divided into two phases: (1) a planning phase, and (2) a procedure phase. The planning and treatment phases for medical treatment (e.g., microwave ablation) are more fully described in U.S. Published Patent Application Nos. 2014/028196113, entitled PATHWAY PLANNING SYSTEM AND METHOD, filed on March 15, 2013, by Baker and U.S. Patent Application Number 14/753,288 entitled SYSTEM AND METHOD FOR NAVIGATING WITHIN THE LUNG filed on June 29, 2015, by Brown et al..

As described in the above applications the use of pre-procedure images, along with 3D models, particularly where the location if medical device is detected and displayed with reference to these images and models enhances clinicians' understanding about locations of the medical device with respect to internal organs of a patient. While these image displaying modalities are quite useful to show the real time location of the medical device with respect to the internal organs during the navigation within the patient, they are not perfect. One source of error is caused by the respiration of the patient. The physical movement of the lungs can cause movement and changes in the physiology of the patient as the lungs inflate and deflate. As can be appreciated, the pre-procedure images are taken at one point in the respiration cycle, often full inspiration while the patient holds their breath. As a result, at all times other than full inspiration (an occurrence which typically does not happen during procedures where the patient is intubated and sedated), there is some error in the registration of the location of the medical device with the pre-procedure images and its actual location within the physiology of the patient. The detected location of the medical device can appear to be moving in the static image or 3D model, and in some cases the detected and displayed movement of the medical device may cause it to appear to be outside a known channel (e.g. lung airway or blood vessel) which the clinician knows is not correct. In accordance with one aspect of the present disclosure, motion sensors which detect the physical movement of the patient are used to stabilize the respiratory-induced movement of the medical device to more accurately reflect the location of the medical device within the patient on the pre-procedure images or 3D model.

In one embodiment, motion sensors may be placed on the chest of the patient and capture respiratory movement. The medical device also includes a sensor, whose motion can be detected. By subtracting the movement of the motion sensors from the sensed movement of the medical device, the apparent movement of the medical device can be greatly reduced, and as a result the movement shown in the images or 3D model is greatly reduced and more accurately reflects the position of the medical device to the patient's physiology.

In a further embodiment, a respiration model may be generated to model the effect of a patient's respiration from the data captured by the motion sensors. Since any internal organ, for example, a portion of the lungs inside the chest moves differently from the respiratory movement of the chest, the respiration model allows for further refinement of the motion to be subtracted from the medical device movement to more accurately identify the position of the medical device with respect to the patient's physiology at locations remote from the locations of the motion sensors.

In the model embodiment, a weight is used to adjust the respiration model to accommodate for distances between the location of the motion sensors and the location of the medical device. The weight is also used to calculate a reference stabilization signal of the medical device, which models changes in location of the medical device due to the respiratory movement. Thus, by subtracting the reference stabilization signal from the detected changes in location of the medical device, which is sensed by a tracking sensor, the discrepancy, which caused by the respiratory movement, between the detected location of the medical device and the location of the medical device with respect to internal organs can be substantially removed and the medical device can be accurately displayed with respect to pre-procedure images or 3D model generated from the pre-procedure images.

Although the present disclosure will be described in terms of specific illustrative embodiments, it will be readily apparent to those skilled in this art that various modifications, rearrangements and substitutions may be made without departing from the spirit of the present disclosure. The scope of the present disclosure is defined by the claims appended hereto.

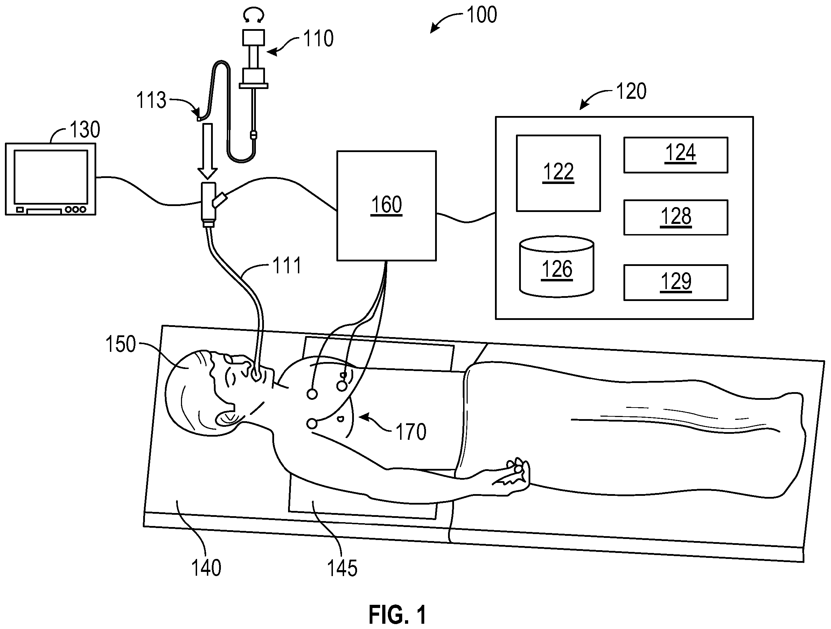

FIG. 1 illustrates an electromagnetic navigation (EMN) system 100 using an electromagnetic field for identifying a real-time location of a medical device within a patient body. The EMN system 100 is configured to augment CT, MRI, or fluoroscopic images, with ultrasound image data assisting in navigation through a luminal network of a patient's lung to a target. One such EMN system 100 may be the ELECTROMAGNETIC NAVIGATION BRONCHOSCOPY® system currently sold by Covidien LP. The EMN system 100 includes a catheter guide assembly 110, a bronchoscope 111, a computing device 120, a monitoring device 130, an EM board 140, an EM tracking system 160, and motion sensor 170. The bronchoscope 111 is operatively coupled to the computing device 120 and the monitoring device 130 via wired connection (as shown in FIG. 1) or wireless connection (not shown).

The computing device 120, such as, a laptop, desktop, tablet, or other similar computing device, includes a display 122, one or more processors 124, memory 126, a network card 128, and an input device 129. The EMN system 100 may also include multiple computing devices, wherein multiple computing devices 120 are employed for planning, treatment, visualization, or helping clinicians in a manner suitable for medical procedures. The display 122 may be touch-sensitive and/or voice-activated, enabling the display 122 to serve as both an input and output device. The display 122 may display a two dimensional (2D) images or 3D models of a chest of the patient to locate and identify a portion of the lung that displays symptoms of lung diseases. The display 122 may further display options to select, add, and remove a target to be treated and settable items for the visualization of the lung. In an aspect, the display 122 may also display the location of the catheter guide assembly 110 in the luminal network of the lung based on the 2D images or 3D model of the chest.

The one or more processors 124 execute computer-executable instructions. The processors 124 may perform image-processing functions so that the 3D model of the lung can be displayed on the display 122. In embodiments, the computing device 120 may further include a separate graphic accelerator (not shown) that performs only the image-processing functions so that the one or more processors 124 may be available for other programs.

The memory 126 stores data and programs. For example, data may be image data for the 3D model or any other related data such as patients' medical records, prescriptions and/or history of the patient's diseases. One type of programs stored in the memory 126 is a 3D model and pathway planning software module (planning software). An example of the 3D model generation and pathway planning software may be the ILOGIC® planning suite currently sold by Medtronic PLC.

The memory 126 may store navigation and procedure software which interfaces with the EMN system 100 to provide guidance to the clinician and provide a representation of the planned pathway on the 3D model and 2D images derived from the 3D model. An example of such navigation software may be the ILOGIC® navigation and procedure suite sold by Covidien LP. In practice, the location of the patient 150 in the EM field generated by the EM field generating device 145 must be registered to the 3D model and the 2D images derived from the model.

The bronchoscope 111 is inserted into the mouth of the patient 150 and captures images of the luminal network of the lung using a video capturing device (not shown). In the EMN system 100, inserted into the bronchoscope 111 is a catheter guide assembly 110 for achieving access to the periphery of the luminal network of the patient 150. The catheter guide assembly 110 may include an extended working channel (EWC) 112 into which a locatable guide catheter (LG) 113 with a tracking sensor 115, which is positioned or integrated near the distal portion of the LG 113, is inserted. The EWC 112, the LG 113, and the tracking sensor 115 are used to navigate through the luminal network of the lung. Though described here with respect to the tracking sensor 115 being included in the LG 113, those of skill in the art will recognize that the tracking sensor 115 may be formed integrally with the EWC 112, or in another component insertable through the EWC 112, such as an ablation catheter, biopsy tool, aspiration needle, tissue piercing and tunneling instrument, and others known to those of skill in the art.

The EM board 140 is configured to provide a flat surface for the patient to lie down and includes an EM field generating device 145. When the patient 150 lies down on the EM board 140, the EM field generating device 145 generates an EM field surrounding a portion of the patient 150. The tracking sensor 115 at the distal portion of the LG 113 is used to determine the location of the EWC 112 in the EM field generated by the EM field generating device 145.

The EM board 140 may be configured to be operatively coupled with the motion sensors 170 which are located around the chest of the patient 150. The motion sensors 170 capture respiratory movement of the chest while the patient 150 is inhaling and exhaling. In an aspect, the motion sensor 170 may be EM sensors configured to sense the strength and changes to the strength of the EM field generated by the EM field generating device 145. Based on the sensed results, locations of the motion sensor 170 may be calculated and thus the patient's respiratory movements are identified.

The tracking sensor 115 and the motion sensors 170 may each be capable of sensing 3 degrees of freedom (DOF) including translational movements along X, Y, and Z axes in the Cartesian coordinate system. The coordinate system may be the polar, spherical, or any suitable coordinate system to represent the EM field space. The tracking sensor 115 and the motion sensor 170 may also be capable of sensing 5 or 6 DOF including the three translational directions and three rotational movements (pitch, yaw, and roll) within the EM field.

While navigating toward a target of interest, movement of the LG 113 (specifically the tracking sensor 115) due to respiration may not manifest itself as significantly on the displayed image of the LG 113 on the pre-procedure images or 3D models as it is initially navigated through the patient. The movement of the LG 113 due to the respiration, however, may affect accuracy, effectiveness, and reliability of the medical procedures including ablation or biopsy when the distal portion of the LG 113 or the EWC 112 approaches a target located closer to the pleura boundaries of the lungs. To address the potential accuracy issues, the EM tracking system 160 receives data representing respiratory movement of the patient's chest as sensed by the motion sensor 170. In an aspect, a respiratory model may be generated by using singular value decomposition or principal component analysis (PCA), as will be described in greater detail below. This respiratory model may refine the data received from the motion sensor 170 to remove portions of a signal received from the motion sensor 170 that is attributable to noise.

By subtracting a reference stabilization signal from the sensed location of the LG 113, a more accurate location (the stabilized location) of the LG 113 with respect to the physiology of a patient, and specifically the internal organs as they move through the respiration process may be obtained. In this way, the sensed location of the LG 113 may be stabilized so that the stabilized location of the LG 113 is synchronized with the respiration cycle of the physiology and the position of the LG 113 is accurately and stably displayed on pre-procedure 2D images or the 3D model.

In an aspect of the present disclosure, a special computer program or software module associated with the EM tracking system 160 may perform procedures and calculations for stabilization based on the respiratory movements.

The positioning of the motion sensor 170 on a patient and the number of the motion sensor 170 affect calculation of a weighting factor and are important in considerations of the present disclosure. As an example, in accordance with aspects of the present disclosure two, three, or more motion sensors 170 may be employed. In at least one embodiment, as shown in FIG. 1, three motion sensors 170 are employed. These three motion sensors 170 are referred to herein as a patient sensor triplet ("PST"). The following description is based on the motion sensors 170 of the PST but the scope of this disclosure is not limited to the three motion sensors. Whether one or more sensor is employed, the sensed movement of the sensors in the EM field is output to generate a respiratory model.

One of the motion sensors 170 of the PST may be placed on the sternum of the patient, specifically about two fingers below the sternal notch. The other two motion sensors 170 of the PST may be placed along left and right sides of the chest, specifically the midaxillary line at the eighth rib on each side. In still another aspect, the placement of the motion sensors 170 of the PST may be determined based on the location of the target of interest so that movements of the LG 113 caused by respiration may be better stabilized with respect to the target.

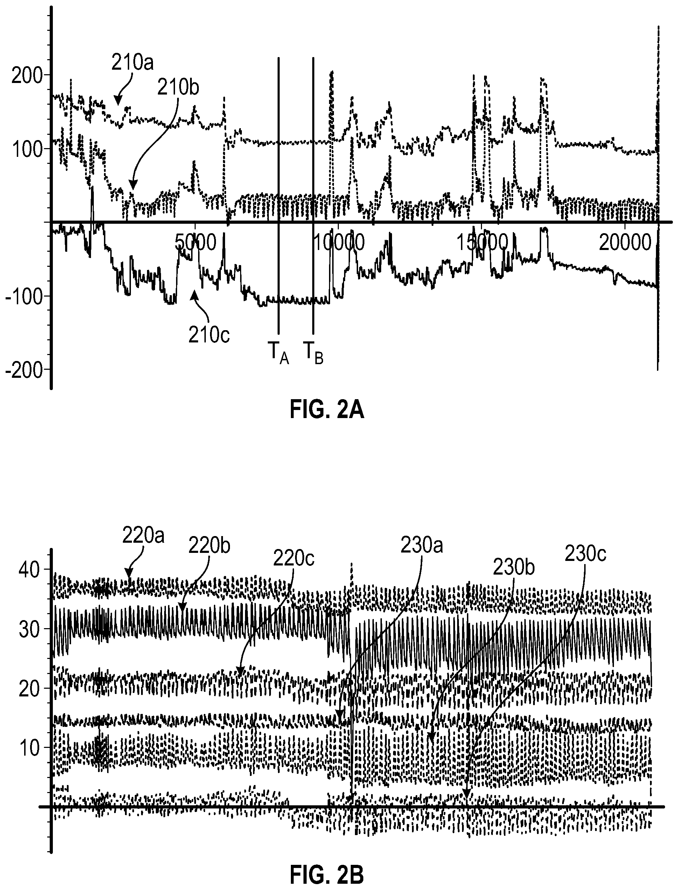

FIG. 2A illustrates graphical representations of sampled movements of the LG 113 (more specifically the tracking sensor 115) due to respiratory movements of the patient. As described above, the tracking sensor 115 located at the distal portion of the LG 113 can sense strength of the EM field in at least three different directions X, Y, and Z. In FIG. 2A, the horizontal axis represents the number of samples taken over time and the vertical axis represents displacement in millimeters (mm) in each of the X, Y and Z directions. In one embodiment of the disclosure, analog-to-digital converters (ADCs), which are not shown in FIG. 1, may capture 30 samples per second in the three directions and a special program or software module installed on the EM tracking system 160 may identify and track the location of the LG 113 in three different directions X, Y, and Z axes over time during the respiration cycle.

Three curves 210a-210c show movement of the tracking sensor 115 at the distal portion of the LG 113 caused by respiration along three different axes (X, Y, and Z). As shown in FIG. 2A, non-periodic displacement of the tracking sensor 115 until time $T_A$ or after $T_B$ represent instances where the tracking sensor 115 of the LG 113 is moved by the clinician. Particularly, non-periodic displacements of the tracking sensor 115 until time $T_A$ may be sampled during navigation toward a target of interest and non-periodic displacements of the tracking sensor 115 until after time $T_B$ may be removal of the LG 113 or re-navigation toward a new target of interest. In contrast, instances the movement is periodically consistent, for example during a period from time $T_A$ to time $T_B$, are likely caused by respiration without movement of the tracking sensor 115 caused by the clinician. This data is the raw movement data of the tracking sensor 115.

In addition to respiratory movement of the lungs, the other organs, such as the heart, or patient's voluntary or involuntary muscle contractions can be detected by the tracking sensor 115. For example, in a case where the LG 113 is placed in proximity to the heart, the position of the LG 113 and the tracking sensor 115 therein will be affected by the movement caused by the heart beating. However, in some instances, while these movements can be detected, their magnitude is sufficiently small that it is desirable to filter these from the detected movement data. Because the frequency of contractions of the heart is higher than respiratory movements and can be easily detected and filtered from the movement data detected by the tracking sensors 115.

When the LG 113 approaches within close proximity to a target or region of interest and an accurate location of the LG 113 is needed, the EM tracking system 160 may manually or automatically start sampling respiratory movements of the chest through the motion sensors 170 of the PST. FIG. 2B illustrates a portion of signals sampled by the ADCs of the EM tracking system 160 from the motion sensors 170 of the PST. The top three curves 220a-220c are movements in the three directions sensed by one motion sensor 170 of the PST and the bottom curves 230a-230c are movements in the three directions sensed by another motion sensor 170 of the PST. Similar signals may be received from the third sensor of the three motion sensors 170 of the PST and additional or fewer motion sensors 170 may be used without departing from the scope of the present disclosure.

In one embodiment, the EM tracking system 160 identifies the respiratory movement of the patient (e.g., the patient's chest) via the motion sensors 170 of the PST for a predetermined period, for example 12-15 seconds, which is sufficient to capture sufficient data for the creation of a respiratory model. The sensed results from the motion sensors 170 of the PST are analyzed to create a respiratory model, which may mimic the patient's respiratory movements but eliminate noise and reduces the number of computations and time necessary to calculate the weights. The respiratory model is derived by performing singular value decomposition or principal component analysis (PCA) on the signals received from the motion sensors 170 of the PST and may be used to reduce the number of parameters for the computations. For example, the ADCs of the EM tracking system 160 may sample 9 signals, 3 signals from each motion sensor 170. A sampling frequency by the ADCs of the EM tracking system 160 may be 30 per second. Thus, for a 15 second sample period, the total number of samples sampled by the ADCs of the EM tracking system 160 is 4050 (9 samples * 30 * 15). In an aspect, by reducing the number of parameters calculation power, time, and resources for performing stabilization calculations based on respiration may be reduced.

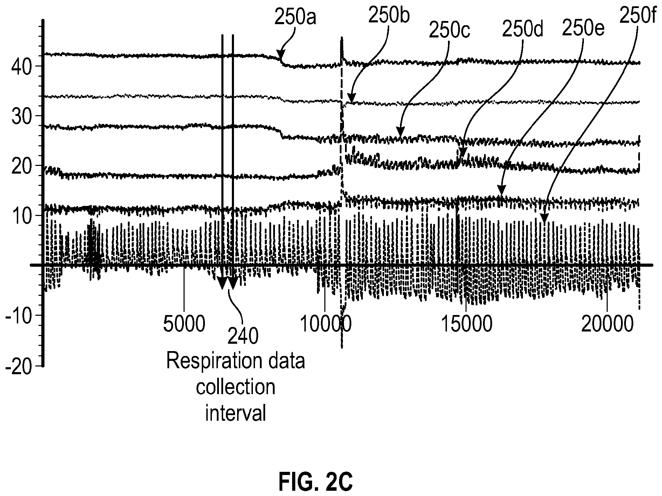

FIG. 2C shows principal components of the signals from the motion sensors 170 using the PCA. The period 240, which is bound by the two vertical lines, is the predetermined sampling period (e.g., 15 seconds). In one example, the predetermined period is longer than or equal to a time required for two consecutive respiration cycles. Other periods and number of respiration cycles may also be utilized without departing from the scope of the present disclosure. For example, the predetermined period may be dependent on requirements of the ADCs of the EM tracking system 160, the motion sensors 170 of the PST, the tracking sensor 115, and others.

In FIG. 2C, curves 250a-250f are illustrative examples of the result of a principal component analysis (PCA) of the signals shown in FIG. 2B. The first principal component 250a may represent the respiratory movements. The second principal component 250b may represent movement based on the heartbeats, and have the second most weight but also includes some noise. The sixth principal component 250f may essentially all noise. The PCA is described in greater detail below.

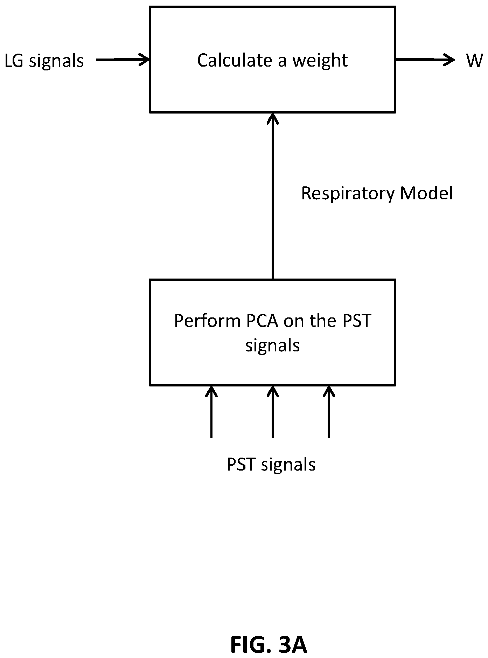

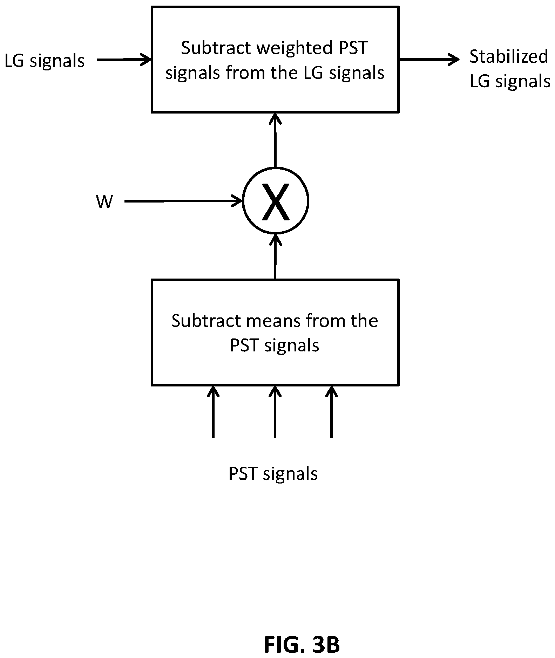

FIG. 3A shows a simplified block diagram illustrating the process of generating weight factor, which will be used to generate a stabilized location signal for the LG 113 using PCA as shown in FIG. 3B. When the LG 113 is placed in close proximity to a target or region of interest, movement of the LG 113 by the clinician is stopped for a predetermined time. During this predetermined time, the ADCs of the EM tracking system 160 sample the respiratory movements of the chest sensed by the motion sensors 170 of the PST and movements of the LG 113 sensed by the tracking sensor 115. During the predetermined period, the clinician does not move the LG 113 and thus the LG 113 maintains its position with respect to the surrounding physiology of the patient. After the predetermined period has passed, a weight factor may be calculated from the samples of the motion sensors 170 of the PST and the LG 113 based on the following equation:

$$ L = AW_1 \tag{1}, $$

where L is an N by 3 matrix having $(X_j, Y_j, Z_j)$ as a row vector sampled by the tracking sensor 115 of the LG 113; A is an N by 9 matrix having $(X_{1j}, Y_{1j}, Z_{1j}, X_{2j}, Y_{2j}, Z_{2j}, X_{3j}, Y_{3j}, Z_{3j})$ as a row vector sampled from motion sensors 170 of the PST, where $(X_{1j}, Y_{1j}, Z_{1j})$, $(X_{2j}, Y_{2j}, Z_{2j})$, and $(X_{3j}, Y_{3j}, Z_{3j})$ are the j-th location sampled from the first, second, and third motion sensors 170 of the PST, respectively, along the X, Y, and Z axes; $W_1$ is an 9 by 3 matrix representing a weight for stabilization based on respiratory movements; and N is the total number of samples collected by each sensor over the predetermined period. The weight $W_1$ may be used to generate a reference stabilization signal for the LG 113 based on sensed movements of the motion sensors 170 of the PST. The reference stabilization signal represents a predicted displacement in the location of the LG 113 due to the respiratory movements and is subtracted from detected LG 113 position to determine a stabilized LG position signal.

As is apparent, A is not a square matrix and its inverse cannot be obtained to calculate the weight $W_1$. In this regard, the EM tracking system 160 may employ PCA, which utilizes the singular value decomposition, to create a respiratory model in matrix representation for A, as will now be described in detail. Referring again to FIG. 3A, upon receipt of the sampled outputs from the motion sensors 170 of the PST, a program or software module installed in the EM tracking system 160 performs PCA by first subtracting the mean of these signals from the signals as follows:

$$ x_{ij} = X_{ij} - \overline{X}_i, \text{ where } \overline{X}_i = \frac{\sum_{j=1}^{N} X_{ij}}{N} \tag{2}, $$

$$ y_{ij} = Y_{ij} - \overline{Y}_i, \text{ where } \overline{Y}_i = \frac{\sum_{j=1}^{N} Y_{ij}}{N} \tag{3}, $$

and

$$ z_{ij} = Z_{ij} - \overline{Z}_i, \text{ where } \overline{Z}_i = \frac{\sum_{j=1}^{N} Z_{ij}}{N} \tag{4}, $$

where i = 1, 2, and 3. Now, a singular value decomposition is applied to the matrix having the mean subtracted for each motion sensor 170 of the PST, as follows:

$$ USV^T = M = \begin{bmatrix} x_{1j} & y_{1j} & z_{1j} & x_{2j} & y_{2j} & z_{2j} & x_{3j} & y_{3j} & z_{3j} \end{bmatrix} \tag{5}, $$

where M is an N by 9 matrix, rows of M include 9 signals from the motion sensors 170 of the PST, columns of U are orthonormal eigenvectors of $MM^T$, columns of V are orthonormal eigenvectors of $M^TM$, and S is an N by 9 matrix containing the squared roots of eigenvalues in the diagonal from U and V in descending order. U is an N by N square matrix and V is a 9 by 9 square matrix. Each entry in the diagonal of S is called a singular value or a principal component. Since diagonal entries of S are in the descending order, the first principal component has the largest value and has the most weight, meaning that the first diagonal entry or the first singular value has the largest effect on M and the other diagonal entries have less effect on M than the first singular value. All entries of S other than the entries in the diagonal are zeros. Now, a respiratory model M is created in matrix representation as $USV^T$, which can be used to calculate the weight $W_1$ as follows:

$$W_1 = VS^{-1}U^TL \qquad (6),$$

where $S^{-1}$ includes entries in the diagonal, which are reciprocals of the non-zero entries in the diagonal of S, and zeros for all other entries.

As noted above, the size of the each matrix defines a large data set meaning that without some reduction in the volume of data the calculation power, processing resources, and time required to calculate the weight $W_1$ would potentially render the methods describe herein too costly or too time consuming to be effective. To address the volume of data issue, the number of principal components can be reduced by removing insignificant principal components so that the number of necessary computations is correspondingly reduced. The present disclosure, however, is not limited to the use of the PCA, and other methodologies for reduction of the dataset or computations may be understood and employed by those of ordinary skill in the art.

By selecting one or more of the nine principal components and zeroing out the rest or replacing them with zeros, time, resources, and power for calculating the weight $W_1$ can be further reduced. For example, the first three principal components, which are the largest of the principal components, may be selected from S to form a new matrix $\tilde{S}$, which includes all zeros other than the three selected singular values. With this new matrix $\tilde{S}$, a respiratory model $\tilde{M}$ can be constructed as follows:

$$\tilde{M} = U\tilde{S}V^T \qquad (7),$$

where $\tilde{S}$ includes the selected principal components in the diagonal and zeros for the other entries. This respiratory model $\tilde{M}$ better represents the respiratory movements of the chest due to the removal of noise-related principal components. With this respiratory model $\tilde{M}$, a weight $W_2$ may be calculated as follows:

$$W_2 = V\tilde{S}^{-1}U^TL \qquad (8),$$

where $\tilde{S}^{-1}$ includes entries in the diagonal, which are reciprocals of the non-zero entries, the selected principal components, in the diagonal of $\tilde{S}$, and zeros for all other entries. Since most of entries of $\tilde{S}^{-1}$ are zero except the number of the selected principal components, calculations for the weight $W_2$ are simpler than calculations of the above equation (6) $W_1 = VS^{-1}U^TL$. In this way, the weight $W_2$ is calculated by the program or software module of the EM tracking system 160. The weight $W_2$ is a 9 by 3 matrix.

After calculating the weight $W_1$ or $W_2$ (hereinafter the weight W), stabilization of the location of the LG 113 with respect to the respiratory movement is performed as shown in FIG. 3B. The ADCs of the EM tracking system 160 sample outputs of the motion sensors 170 of the PST and outputs of the tracking sensor 115 of the LG 113, respectively, at every sampling time for stabilization. The means of the 9 locations from the motion sensors 170 of the PST are subtracted from the 9 location values from the motion sensors 170 of the PST and are multiplied by the weight W to obtain a reference stabilization signal. Specifically, the reference stabilization signal is calculated using the following equation:

$$R = \begin{bmatrix} X_1 & Y_1 & Z_1 & X_2 & Y_2 & Z_2 & X_3 & Y_3 & Z_3 \end{bmatrix} W \qquad (9),$$

where $(X_1, Y_1, Z_1)$, $(X_2, Y_2, Z_2)$, and $(X_3, Y_3, Z_3)$ are sampled locations from the first, second, and third motion sensors 170 of the PST, respectively, along the X, Y, and Z axes; and R is the reference stabilization signal and a 1 by 3 matrix, which represents a predicted displacement for the LG 113 based on the respiratory model. The reference stabilization signal R is subtracted from the tracking sensor 115 signal, and results in a stabilized location of the LG 113. As a result, a graphical representation of the LG 113 at the stabilized location, which is displayed over the pre-procedure 2D images or 3D model on the display, will not noticeably move with respect to the pre-procedure 2D images or 3D model on the display 122.

In an embodiment, a weight may be calculated for each motion sensor 170 of the PST satisfying the following formula:

$$L = M_i W_i = [x_i \ y_i \ z_i] W_i \qquad (10),$$

where M; is a N by 3 matrix or each row $[x_i, y_i, z_i]$ of $A_i$, which is a mean subtracted signal from the i-th motion sensor 170 of the PST, $W_i$ is the weight corresponding to the i-th motion sensor 170, and i is 1, 2, and 3. The weight $W_i$ can be calculated by performing PCA, during which a respiratory model may be generated by selecting a portion of the principal components. Descriptions for detailed procedures for performing PCA have been described above and are omitted here. The reference stabilization signal R may be calculated as follows:

$$R = \frac{\sum_{i=1}^{3} [X_i \ Y_i \ Z_i] W_i}{3} \qquad (11).$$

The stabilized location of the LG 113 is calculated by subtracting the reference stabilization signal R from the sensed location of the tracking sensor 115.

In another embodiment, differences between samples of the motion sensors 170 of the PST may be used to generate the weight. Patients under medical procedures can move voluntarily or involuntarily. Such movements may be shown in the samples collected by the motion sensors 170 of the PST and by the tracking sensor 115 as common-mode shifts. By taking differences between samples, the common-mode shifts may be removed. The singular value decomposition is applied to a difference matrix D as follows:

$$U_D S_D V_D^T = D = [x_{1j} - x_{2j} \ y_{1j} - y_{2j} \ z_{1j} - z_{2j} \ x_{2j} - x_{3j} \ y_{2j} - y_{3j} \ z_{2j} - z_{3j}] \quad (12),$$

where $x_{ij}$, $y_{ij}$, and $z_{ij}$ are mean subtracted signals from the motion sensors 170 of the PST, and $U_D$, $S_D$, $V_D$ are corresponding matrixes to the difference matrix based on the singular value decomposition. $U_D S_D V_D^T$ is a respiratory model for the difference matrix D and is used to calculate a weight $W_D$ by the following equation:

$$W_D = V_D S_D^{-1} U_D^T L \qquad (13).$$

After the predetermined period, a reference stabilization signal $R_D$ for the LG 113 based on the difference matrix D is calculated as follows:

$$R_D = [X_1 - X_2 \ Y_1 - Y_2 \ Z_1 - Z_2 \ X_2 - X_3 \ Y_2 - Y_3 \ Z_2 - Z_3] W_D \qquad (14).$$

Since the total number of singular values is six when using the difference matrix D, calculation power, time, and resources may also be reduced. In the same way described above, a portion of the principal components of $S_D$ may be selected for constructing a respiratory model and calculating the weight $W_D$ with the respiratory model so as to further reduce calculation power, time, and resources.

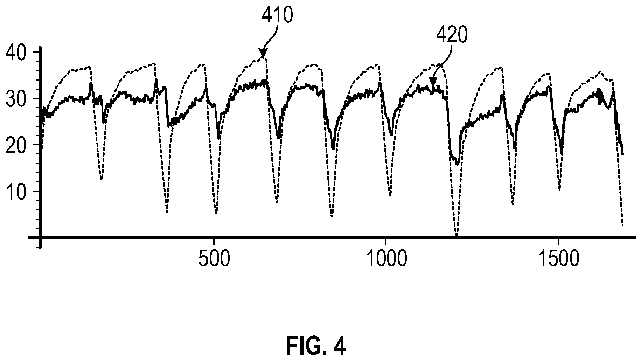

FIG. 4 shows locations of the LG 113 before and after the stabilization based on the respiratory movements. Curve 410 illustrates movement (i.e., location over time) of the LG 113 in one axis (e.g., the X-axis) before the stabilization. As shown in the curve 410, even though the LG 113 is not moved to navigate toward a target, the effects of respiratory movements are apparent. Displacement of the location of the LG 113 may be as much as 4 centimeters in one direction (X, Y. or Z axis) and is also shown in the curve 210b of FIG. 2A during the period from time $T_A$ to time $T_B$.

Curve 420 illustrates the stabilized movement of the LG 113. As compared to the displacement of the LG 113 before stabilization, the maximum displacement in the stabilized movement signal is less than about 1 centimeter even at instances of maximum displacement.

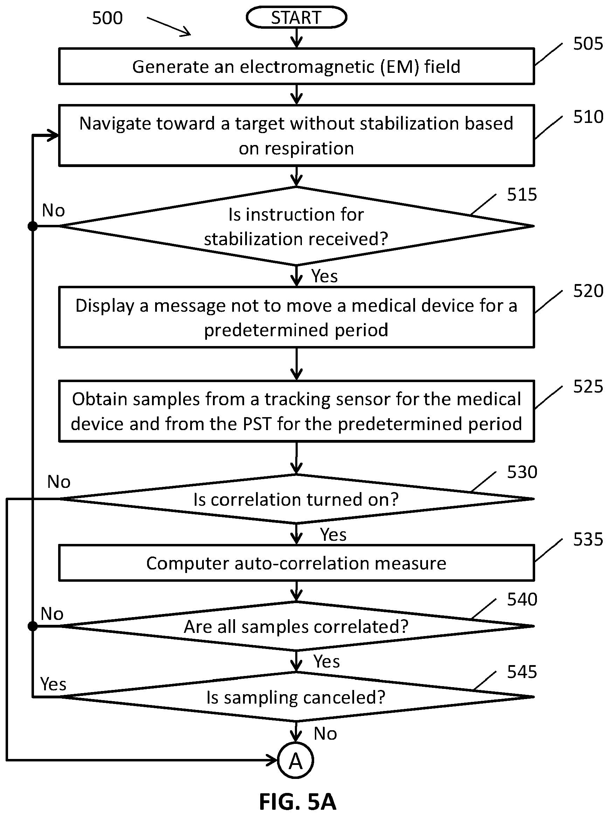

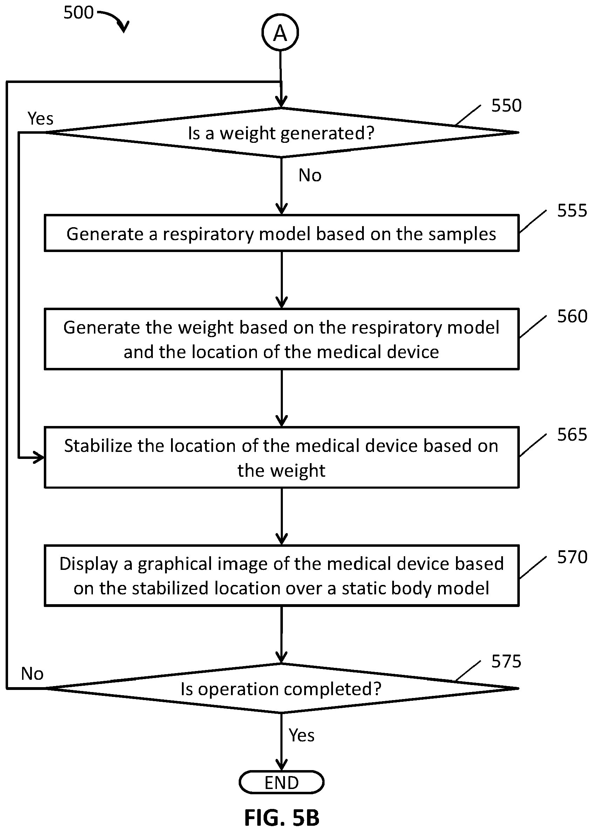

FIGS. 5A and 5B are flow charts illustrating a method 500 for manually stabilizing the respiratory movements for the LG 113 in accordance with embodiments of the present disclosure. When a patient is placed on the EM board 140, the method 500 is started by generating an EM field at step 505, for example using the EM field generating device 145.

At step 510, a clinician follows a pathway plan for navigation within the luminal structure of the patient (e.g. the airways of the lungs) so that the LG 113 navigates toward a target. At this stage, displacement of locations of the LG 113 caused by respiratory movements may be minimal in comparison to the movement caused by advancement of the LG 113 by the clinician, and thus respiration-induced movements can be ignored. At step 515, it is determined whether an instruction for stabilization is received. In at least one embodiment, this may be instituted by the clinician by clicking of a button on a user interface of a procedure software application presented on display 122. If not, the clinician continues navigation of the LG 113.

When it is determined that the instruction is received in step 515, the EM tracking system 160 then displays a message on the display 122, warning that the LG 113 should not be moved for a predetermined period at step 520. In some, though not necessarily all instances, it will be understood that receipt of the instruction to initiate stabilization based on respiration occurs when the LG 113 is in close proximity to the target where displacement of the location of the LG 113 caused by the respiratory movement may have significant effect on the following steps of a medical procedure. In an aspect, the predetermined period may be greater than or equal to a period for at least two consecutive respiration cycles. The warning message may be a textual message displayed via a user interface on the display 122 or an audio message.

During the predetermined period, the EM tracking system 160 obtains samples from the tracking sensor 115 for locations of the LG 113, and samples from the motion sensors 170 of the PST at step 525.

At step 530, a determination must be made whether it is determined whether a correlation function is turned on. Correlation of the sampled data may be used to determine whether the obtained samples show periodic displacements in any direction. In other words, the correlation can be another safety feature ensuring that displacements identified from the obtained samples are caused mainly by the respiratory movements and can be used for stabilization.

When it is determined that the correlation is turned on, an auto-correlation measure is computed in step 535. As described above, during the predetermined period, the LG 113 is not to be moved by the clinician. This auto-correlation measure is used to check whether periodic displacements are shown in the obtained samples during the predetermined period by the tracking sensor 115 of the LG 113. For example, referring back to FIG. 2A, samples obtained during a period from time $T_A$ to time $T_B$ show periodic displacements, which can be identified by the auto-correlation measure. In contrast, samples obtained during a period until time $T_A$ or after time $T_B$ do not show periodic displacements for the predetermined time, which can be also identified by the auto-correlation measure. Thus, the auto-correlation measure is used in step 540 to check whether stabilization based on the respiratory movement can be started. If it is determined that the auto-correlation measure is less than or equal to a threshold or the auto-correlation measure indicates that periodic movement exists in the obtained samples, the method 500 goes back to step 510.

When it is determined that the correlation is not turned on in step 530 or when it is determined that all signals are correlated at step 540, a further step 545 is to determine whether sampling has been cancelled. This may be performed by selection of a stop sampling button on a user interface by a clinician or automatically when it is determined that the sensed motion of the LG 113 are not caused by respiration but by other causes, such as further navigation of the LG 113 within the patient. If the sampling is canceled, stabilization based on the respiratory movements cannot be performed and the method 500 goes back to step 510.

When it is determined that sampling has not been canceled in step 545, the method progresses to step 550 where it is determined whether a weight factor has already been calculated. If the weight factor has been already calculated, then no new weight needs to be calculated and the method 500 proceeds to step 565. If no weight factor has been calculated, the EM tracking system 160 generates a respiratory model of the chest in step 555 based on the samples obtained by the motion sensors 170 of the PST.

As described above in FIG. 3A, a few principal components may be selected for the respiratory model based on PCA or other suitable methodologies to reduce computational power, time, and resources and to better represent the respiration-induced movements by removing other periodic or non-periodic movements. In an aspect, the number of selected principal components may be dependent upon a threshold. For example, if the threshold is 90 percent, the largest principal component is selected until the sum of the selected principal components is greater than or equal to 90 percentage of the total sum of all the principal components. The respiratory model $\tilde{A}$ may be obtained after selecting the largest principal components from the following equation:

$$\tilde{A} = U\tilde{S}V^{T} \tag{15},$$

where $\tilde{S}$ only includes the selected principal components in the diagonal and all zeros for the other entries. Detailed descriptions for the singular value decomposition have been described with respect to FIG. 3A above and are omitted here.

In step 560, the weight W is calculated based on the respiratory model and the samples obtained from the tracking sensor 115 for the locations of the LG 113, based on equation (6), (8), (10), or (13) above. Once the weight W is calculated after the predetermined period, stabilization can be initiated. At step 565, new samples obtained from the motion sensors 170 of the PST at every sampling time for stabilization after the predetermined period are multiplied by the weight W to generate a reference stabilization signal for the LG 113. The reference stabilization signal is subtracted from new location data of the tracking sensor 115 at the same sampling time to generate a stabilized location of the LG 113. Once the weight W is calculated for the target of interest, the same weight is used to stabilize the location of the LG 113 during a medical operation for the same target. Thus, calculations for the stabilized location of the LG 113 are simple and thus can be performed real-time.

In an aspect, at step 565, the weight may be updated based on changes to the locations of the LG 113 with respect to the motion sensors 170 of the PST. For example, a new weight may be calculated for every predetermined period (e.g., two consecutive respiration cycles) and a weighted average between the previous weight and the new weight may be calculated as the updated weight. By updating the weight, abrupt changes in samples from the motion sensors 170 of the PST or from the tracking sensor 115 may be subdued.

At step 570, the EM tracking system 160 then displays a graphical representation of the LG 113 on the display 122 based on the stabilized location with reference to the pre-procedure 2D images or the 3D model. The displayed stabilized location minimizes the effect of breathing on the display of the detected location of the LG 113, and the clinician is provided greater accuracy with respect to the actual physiology proximate the LG 113 being shown in the display 122 and the medical procedures may be performed with greater accuracy than without stabilization based on the respiratory movements.

It is determined whether the medical procedure is completed for the target of interest at step 575. If it is determined that the procedure is not completed, the stabilization based on the respiratory movements continues until the medical procedure for the target of interest is determined complete by reiterating steps 550-575. When it is determined that the medical procedure is completed, the method 500 ends for the target of interest. In an aspect, if there is another target of interest for medical procedures, method 500 is restarted and performed until the medical procedure for the new target is completed.

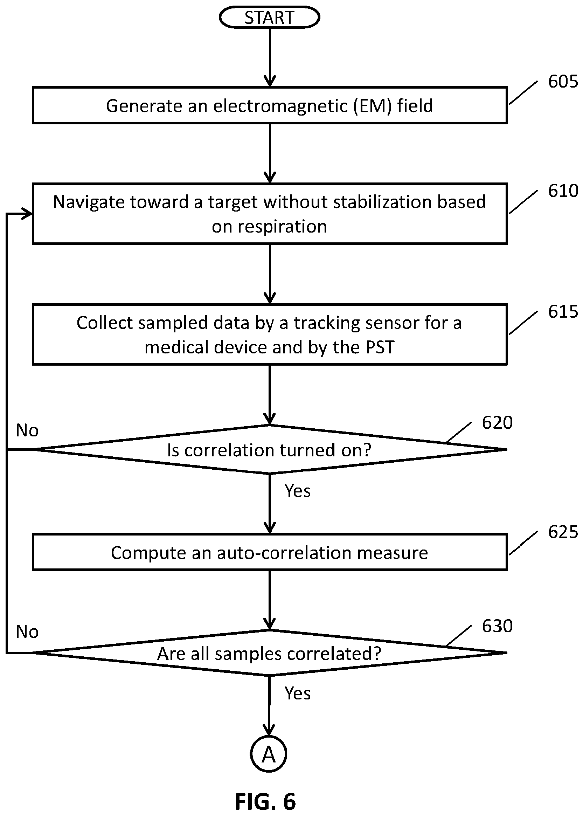

FIG. 6 shows a flow chart illustrating a method 600 for automatically stabilization based on the respiratory movements for the LG 113 in accordance with an embodiment of the present disclosure. As in FIG. 5A, this method also starts with generating an EM field at step 605. At step 610, a clinician follows a pathway plan so that the LG 113 navigates toward a target of interest without stabilization based on the respiratory movements.

At step 615, the ADCs of the EM tracking system 160 samples data from the tracking sensor 115 for the LG 113 and from the motion sensors 170 of the PST. At step 620, it is determined whether the correlation is turned on. The method 600 goes back to step 610 when the correlation is determined not being turned on.

When it is determined that the correlation is turned on, the EM tracking system 160 calculates an auto-correlation measure based on the samples from the motion sensors 170 of the PST and the tracking sensor 115 at step 625. The auto correlation measure has been described in FIG. 5A and thus descriptions thereof are omitted here. It is also determined whether all samples are correlated based on the auto-correlation measure at step 630. If it is determined that the all samples are not correlated, the method 600 goes back to step 610. When it is determined that the all samples are correlated or periodic movements are detected in the obtained samples, the method 600 follows the steps 550-575 of FIG. 5B until medical procedure is completed.

Although embodiments have been described in detail with reference to the accompanying drawings for the purpose of illustration and description, it is to be understood that the inventive processes and apparatus are not to be construed as limited thereby. It will be apparent to those of ordinary skill in the art that various modifications to the foregoing embodiments may be made without departing from the scope of the disclosure.

**Claims**

1.  A system (100) for stabilization based on respiratory movement, the system comprising:

    a medical device (113) configured to navigate inside of a patient (150);
    a tracking sensor (115) affixed on the medical device and configured to track a location of the medical device;
    at least one motion sensor (170) adapted to be located on the patient and configured to sense respiratory movements of the patient;
    a computer (120) configured to generate a respiratory model based on the respiratory movements sensed by the at least one motion sensor for a predetermined period, and to stabilize the location of the medical device based on the respiratory model after the predetermined period; and
    a display (122) configured to display a graphical representation of the medical device based on the stabilized location on a pre-procedure two-dimensional (2D) image or three-dimensional (3D) model, **characterized in that**

the computer is further configured to: (i) check an auto-correlation of the tracked locations of the medical device during the predetermined period; (ii) restart the predetermined period if the correlation is greater than a threshold; and (iii) generate a weight factor based on the respiratory model and the tracked locations of the medical device if the correlation is less than or equal to the threshold for the predetermined time period.

2. The system according to claim 1, wherein the computer is further configured to receive an instruction to start sampling outputs of the at least one motion sensor and outputs of the tracking sensor for the predetermined period.

3. The system according to claim 2, wherein the computer is further configured to calculate the weight factor based on the respiratory model and the tracked locations of the medical device, which have been sampled for the predetermined period.

4. The system according to claim 3, wherein a new location of the medical device and a new respiratory movement of the at least one motion sensor are sampled at each sampling time for stabilization after the predetermined period.

5. The system according to claim 4, wherein the computer is further configured to multiply the new respiratory movement from the motion sensor with the weight factor to obtain a reference stabilization signal.

6. The system according to claim 5, wherein the stabilized location of the medical device is obtained by subtracting the reference stabilization signal from the new location of the medical device.

7. The system according to claim 2, wherein the predetermined period is at least two consecutive respiratory cycles.

8. The system according to claim 2, wherein the respiratory model is based on mean subtracted sampled outputs of the at least one motion sensor for the predetermined period.

9. The system according to any preceding claim, wherein a new location of the medical device and a new respiratory movement from the at least one motion sensor are sampled at each sampling time for stabilization after the predetermined period.

10. The system according to claim 9, wherein the computer is further configured to multiply the new respiratory movement from the at least one motion sensor with the weight factor to obtain a reference stabilization signal for the medical device.

11. The system according to claim 10, wherein the stabilized location of the medical device is obtained by subtracting the reference stabilization signal from the new location of the medical device.

12. The system according to any preceding claim, wherein the tracked locations of the medical device are correlated when a periodic movement exists in the tracked locations of the medical device, which have been sampled during the predetermined period.

**Patentansprüche**

1. System (100) für eine Stabilisierung basierend auf Atembewegungen, wobei das System Folgendes umfasst:

eine medizinische Vorrichtung (113), die konfiguriert ist, um innerhalb eines Patienten (150) zu navigieren;
einen Verfolgungssensor (115), der an der medizinischen Vorrichtung angebracht und konfiguriert ist, um eine Position der medizinischen Vorrichtung zu verfolgen;
wenigstens einen Bewegungssensor (170), der angepasst ist, um sich an dem Patienten zu befinden und konfiguriert ist, um Atembewegungen des Patienten zu erfassen;
einen Computer (120), der konfiguriert ist, um ein Atemmodell basierend auf den Atembewegungen, die durch den wenigstens einen Bewegungssensor für eine zuvor bestimmte Zeitdauer erfasst werden, zu erzeugen und um die Position der medizinischen Vorrichtung basierend auf dem Atemmodell nach der zuvor bestimmten Zeitdauer zu stabilisieren; und
eine Anzeige (122), die konfiguriert ist, um eine grafische Darstellung der medizinischen Vorrichtung basierend auf der stabilisierten Position auf einem zweidimensionalen (2D) Bild vor dem Eingriff oder einem dreidimensionalen (3D) Modell anzuzeigen, **dadurch gekennzeichnet, dass**

der Computer ferner für Folgendes konfiguriert ist: (i) Überprüfen einer Autokorrelation der verfolgten Positionen der medizinischen Vorrichtung während der zuvor bestimmten Zeitdauer; (ii) Neustarten der zuvor bestimmten Zeitdauer, falls die Korrelation größer als ein Schwellenwert ist; und (iii) Erzeugen eines Gewichtsfaktors basierend auf dem Atemmodell und den verfolgten Positionen der medizinischen Vorrichtung, falls die Korrelation kleiner als oder gleich dem Schwellenwert für die zuvor bestimmte Zeitdauer ist.

2. System nach Anspruch 1, wobei der Computer ferner konfiguriert ist, um eine Anweisung zu empfangen, um ein Abtasten von Ausgaben des wenigstens einen Bewegungssensors und Ausgaben des Verfolgungssensors für den zuvor bestimmten Zeitraum zu starten.

3. System nach Anspruch 2, wobei der Computer ferner konfiguriert ist, um den Gewichtsfaktor basierend auf dem Atemmodell und den verfolgten Positionen der medizinischen Vorrichtung zu berechnen, die über die zuvor bestimmte Zeitdauer abgetastet wurden.

4. System nach Anspruch 3, wobei eine neue Position der medizinischen Vorrichtung und eine neue Atembewegung des wenigstens einen Bewegungssensors zu jedem Abtastzeitpunkt für die Stabilisierung nach der zuvor bestimmten Zeitdauer abgetastet werden.

5. System nach Anspruch 4, wobei der Computer ferner konfiguriert ist, um die neue Atembewegung von dem Bewegungssensor mit dem Gewichtsfaktor zu multiplizieren, um ein Referenzstabilisierungssignal zu erhalten.

6. System nach Anspruch 5, wobei die stabilisierte Position der medizinischen Vorrichtung durch Subtrahieren des Referenzstabilisierungssignals von der neuen Position der medizinischen Vorrichtung erhalten wird.

7. System nach Anspruch 2, wobei die zuvor bestimmte Zeitdauer wenigstens zwei aufeinanderfolgende Atemzyklen beträgt.

8. System nach Anspruch 2, wobei das Atemmodell auf mittleren subtrahierten abgetasteten Ausgaben des wenigstens einen Bewegungssensors für die zuvor bestimmte Zeitdauer basiert.

9. System nach einem der vorhergehenden Ansprüche, wobei eine neue Position der medizinischen Vorrichtung und eine neue Atembewegung von dem wenigstens einen Bewegungssensor zu jedem Abtastzeitpunkt für die Stabilisierung nach der zuvor bestimmten Zeitdauer abgetastet werden.

10. System nach Anspruch 9, wobei der Computer ferner konfiguriert ist, um die neue Atembewegung von dem wenigstens einen Bewegungssensor mit dem Gewichtsfaktor zu multiplizieren, um ein Referenzstabilisierungssignal für die medizinische Vorrichtung zu erhalten.

11. System nach Anspruch 10, wobei die stabilisierte Position der medizinischen Vorrichtung durch Subtrahieren des Referenzstabilisierungssignals von der neuen Position der medizinischen Vorrichtung erhalten wird.

12. System nach einem der vorhergehenden Ansprüche, wobei die verfolgten Positionen der medizinischen Vorrichtung korreliert sind, wenn an den verfolgten Positionen der medizinischen Vorrichtung, die während der zuvor bestimmten Zeitdauer abgetastet wurden, eine periodische Bewegung besteht.

**Revendications**

1. Système (100) de stabilisation basé sur le mouvement respiratoire, le système comprenant :

un dispositif médical (113) configuré pour naviguer à l'intérieur d'un patient (150) ;
un capteur de suivi (115) fixé sur le dispositif médical et configuré pour suivre un emplacement du dispositif médical ;
au moins un capteur de mouvement (170) adapté pour être situé sur le patient et configuré pour détecter les mouvements respiratoires du patient ;
un ordinateur (120) configuré pour générer un modèle respiratoire sur la base des mouvements respiratoires détectés par l'au moins un capteur de mouvement pendant une période prédéterminée, et pour stabiliser l'emplacement du dispositif médical sur la base du modèle respiratoire après la période prédéterminée ; et

un dispositif d'affichage (122) configuré pour afficher une représentation graphique du dispositif médical sur la base de l'emplacement stabilisé sur une image bidimensionnelle (2D) ou un modèle tridimensionnel (3D) de pré-procédure, **caractérisé en ce que**
l'ordinateur est en outre configuré pour : (i) vérifier une auto-corrélation des emplacements suivis du dispositif médical pendant la période prédéterminée ; (ii) redémarrer la période prédéterminée si la corrélation est supérieure à un seuil ; et (iii) générer un facteur de poids sur la base du modèle respiratoire et des emplacements suivis du dispositif médical si la corrélation est inférieure ou égale au seuil pour la période prédéterminée.

2. Système selon la revendication 1, dans lequel l'ordinateur est en outre configuré pour recevoir une instruction afin de démarrer l'échantillonnage des sorties de l'au moins un capteur de mouvement et des sorties du capteur de suivi pour la période prédéterminée.

3. Système selon la revendication 2, dans lequel l'ordinateur est en outre configuré pour calculer le facteur de poids sur la base du modèle respiratoire et des emplacements suivis du dispositif médical, qui ont été échantillonnés pour la période prédéterminée.

4. Système selon la revendication 3, dans lequel un nouvel emplacement du dispositif médical et un nouveau mouvement respiratoire de l'au moins un capteur de mouvement sont échantillonnés à chaque temps d'échantillonnage pour une stabilisation après la période prédéterminée.

5. Système selon la revendication 4, dans lequel l'ordinateur est en outre configuré pour multiplier le nouveau mouvement respiratoire à partir du capteur de mouvement par le facteur de poids pour obtenir un signal de stabilisation de référence.

6. Système selon la revendication 5, dans lequel l'emplacement stabilisé du dispositif médical est obtenu en soustrayant le signal de stabilisation de référence du nouvel emplacement du dispositif médical.

7. Système selon la revendication 2, dans lequel la période prédéterminée est d'au moins deux cycles respiratoires consécutifs.

8. Système selon la revendication 2, dans lequel le modèle respiratoire est basé sur la moyenne des sorties échantillonnées soustraites de l'au moins un capteur de mouvement pour la période prédéterminée.

9. Système selon l'une quelconque des revendications précédentes, dans lequel un nouvel emplacement du dispositif médical et un nouveau mouvement respiratoire à partir de l'au moins un capteur de mouvement sont échantillonnés à chaque temps d'échantillonnage pour une stabilisation après la période prédéterminée.

10. Système selon la revendication 9, dans lequel l'ordinateur est en outre configuré pour multiplier le nouveau mouvement respiratoire à partir de l'au moins un capteur de mouvement par le facteur de poids pour obtenir un signal de stabilisation de référence pour le dispositif médical.

11. Système selon la revendication 10, dans lequel l'emplacement stabilisé du dispositif médical est obtenu en soustrayant le signal de stabilisation de référence à partir du nouvel emplacement du dispositif médical.

12. Système selon l'une quelconque des revendications précédentes, dans lequel les emplacements suivis du dispositif médical sont corrélés lorsqu'un mouvement périodique existe dans les emplacements suivis du dispositif médical, qui ont été échantillonnés pendant la période prédéterminée.

**FIG. 1**

EP 3 506 827 B1

**FIG. 2A**

**FIG. 2B**

FIG. 2C

**FIG. 3A**

LG signals → | Subtract weighted PST signals from the LG signals | → Stabilized LG signals

W → (X)

(X) ← Subtract means from the PST signals

PST signals

**FIG. 3B**

FIG. 4

500

START

Generate an electromagnetic (EM) field — 505

Navigate toward a target without stabilization based on respiration — 510

No ← Is instruction for stabilization received? — 515

Yes

Display a message not to move a medical device for a predetermined period — 520

Obtain samples from a tracking sensor for the medical device and from the PST for the predetermined period — 525

No ← Is correlation turned on? — 530

Yes

Computer auto-correlation measure — 535

No ← Are all samples correlated? — 540

Yes

Yes ← Is sampling canceled? — 545

No

A

**FIG. 5A**

500

(A)

Yes

Is a weight generated? — 550

No

Generate a respiratory model based on the samples — 555

Generate the weight based on the respiratory model and the location of the medical device — 560

Stabilize the location of the medical device based on the weight — 565

Display a graphical image of the medical device based on the stabilized location over a static body model — 570

No

Is operation completed? — 575

Yes

END

**FIG. 5B**

EP 3 506 827 B1

FIG. 6

23

**REFERENCES CITED IN THE DESCRIPTION**

**Patent documents cited in the description**

- US 2009216114 A
- US 2012172702 A
- US 2014028196113 A
- US 75328815